# EUROPEAN PATENT SPECIFICATION

(11) **EP 3 880 171 B1**
(45) Date of publication and mention of the grant of the patent: **07.06.2023**
(21) Application number: 19813181.5
(22) Date of filing: 15.11.2019
(51) Int. Cl.: A61K 9/00, A61K 9/10, A61K 9/14, A61K 9/16, A61K 9/20, A61K 31/192, A61K 47/02

(54) **IBUPROFEN-CONTAINING ORAL PHARMACEUTICAL FORMULATION**
IBUPROFENHALTIGE ORALE PHARMAZEUTISCHE FORMULIERUNG
FORMULATION PHARMACEUTIQUE ORALE CONTENANT DE L'IBUPROFÈNE

(30) Priority: 16.11.2018 JP 2018215891
(43) Date of publication of application: 22.09.2021
(73) Proprietor: SSP Co., Ltd., Tokyo 163-1488 (JP)
(72) Inventor: ONUKI, Yoichi, Narita-shi, Chiba 286-8511 (JP); YOSHIMURA, Satoru, Tokyo 163-1488 (JP)
(74) Representative: Santarelli
(86) International application number: PCT/JP2019/044835
(87) International publication number: WO 2020/101012

(56) References cited:
- EP-A1- 0 264 187
- JP-A- 2007 023 026
- JP-A- 2017 043 546

## Description

### Technical Field

The present invention relates to a method for reducing the unpleasant taste of ibuprofen.

### Background Art

Non-steroidal antipyretic analgesic anti-inflammatory agents are used to alleviate fever and pain; of these, medical drugs that contain ibuprofen (chemical name: 2-(4-isobutylphenyl)propionic acid) are widely prescribed and commercially available. However, although ibuprofen has an excellent anti-inflammatory analgesic effect, it is disadvantageous in that it has an unpleasant taste: it is bitter and irritates mucous membranes, etc. in the mouth when taken orally. Incorporating magnesium chloride has therefore been suggested as a technique for reducing the unpleasant taste of ibuprofen-containing liquid agents (Patent Literature 1). Patent Literature 2 discloses aluminium hydroxide to mask the taste of ibuprofen.

### Citation List

### Patent Literature

PTL 1: Japanese unexamined patent application publication no 2001-31562
PTL 2: EP0264187

### Summary of Invention

### Technical Problem

However, although magnesium chloride can reduce the unpleasant taste to some extent when the ibuprofen is a liquid agent, it cannot sufficiently reduce the unpleasant taste when the ibuprofen is a solid formulation. Moreover, it is also desirable to further reduce the unpleasant taste of ibuprofen-containing liquid agents.

The problem addressed by the present invention is the provision of technology for reducing the unpleasant taste of ibuprofen in ibuprofen-containing oral pharmaceutical formulations.

### Solution to Problem

As a result of diligent research into solving the problem described above, the present inventors perfected the present invention upon discovering that by incorporating ibuprofen or salt thereof or solvate of these, and magnesium oxide, into an oral pharmaceutical formulation, such that the mass ratio [(B)/(A)] of the ibuprofen or salt thereof or solvate of these (A) and the magnesium oxide (B) is 0.2-1.5, it is possible to reduce the unpleasant taste of ibuprofen.

Specifically, the disclosure provides <1> to <17> below, wherein only <14> and <15> are claimed.
<1> An oral pharmaceutical formulation in which the unpleasant taste of ibuprofen is reduced, comprising (A) ibuprofen or salt thereof or solvate of these, and (B) magnesium oxide, wherein the mass ratio [(B)/(A)] of component (A) and component (B) is 0.2-1.5.
<2> The oral pharmaceutical formulation described in <1>, wherein the mass ratio [(B)/(A)] is 0.25-1.5.
<3> The oral pharmaceutical formulation described in <1> or <2>, wherein the mass ratio [(B)/(A)] is 0.3-1.5.
<4> The oral pharmaceutical formulation described in any of <1> to <3>, wherein the mass ratio [(B)/(A)] is 0.4-1.5.
<5> The oral pharmaceutical formulation described in any of <1> to <4>, wherein the dosage form is a liquid formulation.
<6> The oral pharmaceutical formulation described in any of <1> to <4>, wherein the dosage form is a solid formulation or semi-solid formulation.
<7> The oral pharmaceutical formulation described in <6>, wherein the dosage form is granules, fine granules, powder, uncoated tablet, OD tablet, chewable tablet, gum, jelly or gummy.
<8> The oral pharmaceutical formulation described in any of <1> to <7>, wherein the content of component (A) is such that 60-800 mg ibuprofen, calculated as free form, can be administered per dose.
<9> A method for producing the oral pharmaceutical formulation described in any of <1> to <8>, comprising a step of incorporating (A) ibuprofen or salt thereof or solvate of these, and (B) magnesium oxide into the same composition, such that the mass ratio [(B)/(A)] of component (A) and component (B) in the oral pharmaceutical formulation is 0.2-1.5.
<10> The method described in <9>, further comprising a step of drying the composition obtained in the abovementioned step.
<11> A method for producing the oral liquid formulation described in <5>, comprising:
   (step A-1) a step of incorporating (A) ibuprofen or salt thereof or solvate of these, and (B) magnesium oxide into the same composition, such that the mass ratio [(B)/(A)] of component (A) and component (B) in the liquid oral formulation is 0.2-1.5,
   (step A-2) a step of mixing the composition obtained in step A-1 and water, and
   (step A-3) a step of heating the liquid composition obtained in step A-2.
<12> A method for producing the oral liquid formulation described in <5>, comprising:
   (step B-1) a step of incorporating (A) ibuprofen or salt thereof or solvate of these, and (B) magnesium oxide into the same composition, such that the mass ratio [(B)/(A)] of component (A) and component (B) in the liquid oral formulation is 0.2-1.5,
   (step B-2) a step of drying the composition obtained in step B-1,
   (step B-3) a step of mixing the dried substance obtained in step B-2 and water, and
   (step B-4) a step of heating the liquid composition obtained in step B-3.
<13> A method for producing the solid or semi-solid oral formulation described in <6>, comprising:
   (step C-1) a step of incorporating (A) ibuprofen or salt thereof or solvate of these, and (B) magnesium oxide into the same composition, such that the mass ratio [(B)/(A)] of component (A) and component (B) in the solid or semi-solid oral formulation is 0.2-1.5, and
   (step C-2) a step of drying the composition obtained in step C-1.
<14> A method for reducing the unpleasant taste of ibuprofen in oral pharmaceutical formulations that contain ibuprofen or salt thereof or solvate of these, comprising a step of incorporating (A) ibuprofen or salt thereof or solvate of these, and (B) magnesium oxide into the same composition, such that the mass ratio [(B)/(A)] of component (A) and component (B) in the oral pharmaceutical formulation is 0.2-1.5.
<15> The method described in <14>, further comprising a step of drying the composition obtained in the abovementioned step.
<16> A method for inhibiting the phenomenon whereby at the time of producing an oral pharmaceutical formulation containing (A) ibuprofen or salt thereof or solvate of these, and (B) magnesium oxide, the oral pharmaceutical formulation turns black; the method comprising a step of incorporating component (A) and component (B) into the same composition, such that the mass ratio [(B)/(A)] of component (A) and component (B) in the oral pharmaceutical formulation is 0.2-1.5.
<17> The method described in <16>, wherein the oral pharmaceutical formulation is a solid oral formulation.

### Advantageous Effects of Invention

According to the present invention, the unpleasant taste of ibuprofen in ibuprofen-containing oral pharmaceutical formulations can be reduced.

### Detailed Description of the Invention

### <Oral pharmaceutical formulation> (not claimed)

The inventive oral pharmaceutical formulation is an oral pharmaceutical formulation in which the unpleasant taste of ibuprofen is reduced; it contains (A) ibuprofen or salt thereof or solvate of these, and (B) magnesium oxide, where the mass ratio [(B)/(A)] of component (A) and component (B) is 0.2-1.5. Specifically, component (A) and component (B) are contained in one pharmaceutical formulation.

### Component (A)

"Ibuprofen or salt thereof or solvate of these" includes: ibuprofen; alkali metal salts of ibuprofen, such as ibuprofen sodium and ibuprofen potassium; and hydrates and alcoholates of these.

The ibuprofen or salt thereof or solvate of these is preferably a powder. The mean particle size of said powder is preferably 5-75 µm, more preferably 15-60 µm. The mean particle size of the powder can be measured by laser diffraction.

The ibuprofen or salt thereof or solvate of these can be produced by a known method, or a commercial product can be used.

From the viewpoint of reducing the unpleasant taste and inhibiting discoloration in the production step, the amount of ibuprofen or salt thereof or solvate of these with respect to the total mass of the inventive oral pharmaceutical formulation is preferably 1-85% by mass, more preferably 2.5-65% by mass, even more preferably 2.5-60% by mass, and even more preferably 4-50% by mass. If the inventive oral pharmaceutical formulation is a solid formulation or semi-solid formulation, from the viewpoint of reducing the unpleasant taste and inhibiting discoloration in the production step it is even more preferably 5-50% by mass, even more preferably 15-50% by mass, and particularly preferably 20-50% by mass. However, if the inventive oral pharmaceutical formulation is a liquid formulation, from the viewpoint of reducing the unpleasant taste it is particularly preferably 4-10% by mass.

### Component (B)

The magnesium oxide may be either light magnesium oxide or heavy magnesium oxide. There are no particular limitations regarding the particle size, specific volume, etc. of the magnesium oxide; the specific volume is preferably 1-12 mL/g, more preferably 2-10 mL/g.

The magnesium oxide can be produced by a known method, or a commercial product can be used. It should be noted that magnesium oxide-containing substances such as synthetic hydrotalcite and sucralfate may be used.

From the viewpoint of reducing the unpleasant taste and inhibiting discoloration in the production step, the magnesium oxide content with respect to the total mass of the inventive oral pharmaceutical formulation is preferably 0.1-95% by mass, more preferably 0.25-85% by mass, even more preferably 0.5-75% by mass, and even more preferably 1-65% by mass. If the inventive oral pharmaceutical formulation is a solid formulation or semi-solid formulation, from the viewpoint of reducing the unpleasant taste and inhibiting discoloration in the production step it is even more preferably 1-55% by mass, and particularly preferably 1-40% by mass; if the inventive oral pharmaceutical formulation is a liquid formulation, from the viewpoint of reducing the unpleasant taste it is even more preferably 1-15% by mass, and particularly preferably 1-7.5% by mass.

The inventive oral pharmaceutical formulation contains component (A) and component (B) at a mass ratio [(B)/(A)] of 0.2-1.5. If the mass ratio [(B)/(A)] is less than 0.2, the unpleasant taste of the ibuprofen is not sufficiently reduced. If the mass ratio [(B)/(A)] in a solid oral formulation is more than 1.5, a phenomenon readily occurs whereby the solid oral formulation turns black at the time of production.

From the viewpoint of reducing the unpleasant taste and inhibiting discoloration in the production step, the mass ratio [(B)/(A)] is preferably no lower than 0.25, more preferably no lower than 0.3, even more preferably no lower than 0.35, and particularly preferably no lower than 0.4; also, from the viewpoint of inhibiting the phenomenon whereby the formulation turns black in the production step, it is preferably no higher than 1.45, more preferably no higher than 1.25.

The unpleasant taste of ibuprofen is further reduced if the mass ratio [(B)/(A)] is 0.25-1. 5, more preferably 0.3-1.5, even more preferably 0.35-1.5, even more preferably 0.4-1.5, even more preferably 0.4-1.45, and particularly preferably 0.4-1.25.

The inventive oral pharmaceutical formulation may also contain drugs other than those described above, depending on the purpose of the formulation. Examples of such drugs include antacids (excluding magnesium oxide), anti-inflammatory agents (excluding ibuprofen), hypnotic sedatives, antitussive expectorants, caffeines, antihistamines, antiallergic agents, anticholinergics, vitamins, muscle relaxants, crude drugs, etc. These may be used singly or in combination of two or more kinds.

Examples of said antacids include sodium bicarbonate, precipitated calcium carbonate, calcium silicate, synthetic aluminum silicate, calcium hydrogen phosphate, anhydrous calcium hydrogen phosphate, aluminum hydroxide gel, dry aluminum hydroxide gel, aluminum hydroxide-sodium hydrogen carbonate co-precipitation product, aluminum hydroxide-magnesium carbonate mixed dry gel, aluminum hydroxide-magnesium carbonate-calcium carbonate co-precipitation product, magnesium hydroxide-potassium aluminum sulfate co-precipitation product, glycine, magnesium silicate, dihydroxyaluminum aminoacetate, magnesium carbonate, etc. These may be used singly or in combination of two or more kinds.

The amount of said antacid with respect to the total mass of the inventive oral pharmaceutical formulation is preferably 0-5% by mass, more preferably 0-1% by mass, even more preferably 0-0.1% by mass, and particularly preferably 0-0.01% by mass.

Examples of said anti-inflammatory agents include glycyrrhizic acid and salts thereof, tranexamic acid, glycyrrhetinic acid, sodium azulene sulfonate, aspirin, salicylamide, etc. These may be used singly or in combination of two or more kinds.

The amount of said anti-inflammatory agent with respect to the total mass of the inventive oral pharmaceutical formulation is preferably 0-32% by mass, more preferably 0-25% by mass.

Examples of said hypnotic sedatives include allylisopropylacetylurea, bromovalerylurea, etc. These may be used singly or in combination of two or more kinds.

The amount of said hypnotic sedative with respect to the total mass of the inventive oral pharmaceutical formulation is preferably 0-42% by mass, more preferably 0-30% by mass.

Examples of said antitussive expectorants include ambroxol hydrochloride, ethyl L-cysteine hydrochloride, potassium guaiacolsulfonate, potassium cresol sulfonate, guaifenesin, bromhexine hydrochloride, L-carbocisteine, codeine phosphate hydrate, dihydrocodeine phosphate, tipepidine citrate, tipepidine hibenzate, dextromethorphan hydrobromide hydrate, dextromethorphan phenolphthalinate, dimemorphan phosphate, noscapine, noscapine hydrochloride hydrate, dl-methylephedrine hydrochloride, dl-methylephedrine saccharin salt, etc. These may be used singly or in combination of two or more kinds.

The amount of said antitussive expectorant with respect to the total mass of the inventive oral pharmaceutical formulation is preferably 0-56% by mass, more preferably 0-40% by mass.

Examples of said caffeines include caffeine hydrate, anhydrous caffeine, caffeinesodium benzoate, etc. These may be used singly or in combination of two or more kinds.

The amount of said caffeine with respect to the total mass of the inventive oral pharmaceutical formulation is preferably 0-28% by mass, more preferably 0-20% by mass.

Examples of said antihistamines and antiallergic agents include mequitazine, azelastine hydrochloride, fexofenadine hydrochloride, epinastine hydrochloride, loratadine, cetirizine hydrochloride, olopatadine hydrochloride, alimemazine tartrate, carbinoxamine maleate, clemastine fumarate, d-chlorpheniramine maleate, dlchlorpheniramine maleate, diphenhydramine hydrochloride, etc. These may be used singly or in combination of two or more kinds.

Examples of said anticholinergics include isopropamide iodide, scopolia extract, scopolia root, belladonna total alkaloid, scopolamine hydrobromide, butylscopolamine bromide, methylbenactyzium bromide, timepidium bromide, pirenzepine, etc. These may be used singly or in combination of two or more kinds.

Examples of said vitamins include vitamin B1, vitamin B1 derivatives, vitamin B2, vitamin B2 derivatives, vitamin C, vitamin C derivatives, hesperidin, hesperidin derivatives, and salts thereof, etc. These may be used singly or in combination of two or more kinds.

Examples of said crude drugs include jiryuu [Pheretima aspergillum], cinnamon, glycyrrhiza, peony, moutan bark, citrus unshiu peel, ginger, sanshou [Zanthoxylum piperitum], jiegeng [Platycodon], ephedra, apricot, Pinellia ternata, shazenso [Plantago asiatica], senega, saiko [Bupleurum falcatum], magnolia flower, etc. These may be used singly or in combination of two or more kinds.

Examples of said muscle relaxants include methocarbamol, chlorzoxazone, pridinol mesylate, chlorphenesin carbamate, eperisone hydrochloride, afloqualone, tizanidine hydrochloride, etc. These may be used singly or in combination of two or more kinds.

The inventive oral pharmaceutical formulation may also contain pharmaceutical additives. Examples of said pharmaceutical additives include excipients such as lactose, sucrose, glucose, mannitol, sorbitol and xylitol; disintegrants such as carmellose sodium, crospovidone, carboxymethylcellulose calcium, low-substituted hydroxypropyl cellulose (hydroxypropyl content preferably 5-16% by mass), crystalline cellulose and calcium carbonate; binders such as gelatin, sodium alginate, hydroxypropyl cellulose, hydroxypropyl methylcellulose, polyvinylpyrrolidone and carboxymethyl cellulose; lubricants such as magnesium stearate and talc; and fluidizers such as light anhydrous silicic acid; etc. These may be used singly or in combination of two or more kinds. Solubilizer, buffer, preservative, perfume, coloring agent, flavoring agent and the like can also be used if necessary.

The total amount of said pharmaceutical additive(s) with respect to the total mass of the inventive oral pharmaceutical formulation is preferably 5-70% by mass, more preferably 10-50% by mass.

It should be noted that if the inventive oral pharmaceutical formulation is a solid formulation or semi-solid formulation, the water content of the oral solid or semi-solid formulation is preferably 0-7% by mass, more preferably 0-5% by mass.

However, if the inventive oral pharmaceutical formulation is a liquid formulation, the water content of the oral liquid formulation is preferably 40-99% by mass, more preferably 60-99% by mass, and particularly preferably 80-99% by mass.

The dosage form of the inventive oral pharmaceutical formulation may be solid formulation, semi-solid formulation or liquid formulation. Of these, solid formulation and semi-solid formulation are preferred. Even when the dosage form of the inventive oral pharmaceutical formulation is a solid formulation or semi-solid formulation, its unpleasant taste is difficult to detect.

Examples of specific liquid formulation dosage forms include elixirs, syrups, suspensions, emulsions, lemonades, etc.

Examples of specific solid formulation and semi-solid formulation dosage forms include granules, fine granules, powders, tablets (uncoated tablets, OD tablets, chewable tablets, dispersible tablets, dissolving tablets, troches, sublingual tablets, buccal tablets, adhesive tablets, effervescent tablets, gums, etc.), pills, capsules (soft capsules, hard capsules, etc.), dry syrups, jellies, gummies, etc. These may be sugar-coated, film-coated or coated with chocolate or the like, in accordance with a known method.

Of the specific solid formulation and semi-solid formulation dosage forms, granules, fine granules, powders, uncoated tablets, OD tablets, chewable tablets, gums, jellies and gummies are preferred; granules, fine granules, uncoated tablets, OD tablets, chewable tablets, jellies and gummies are more preferred; granules, fine granules, uncoated tablets, OD tablets, chewable tablets and gummies are even more preferred; and granules, uncoated tablets, OD tablets, chewable tablets and gummies are particularly preferred. The mean particle size of the granules is preferably 50-1000 µm, more preferably 50-500 µm. Said mean particle size can be measured by the sieving method.

The present invention can sufficiently reduce the unpleasant taste of ibuprofen, even in types of dosage form that readily impart the unpleasant taste of ibuprofen, such as granules, fine granules, powders, uncoated tablets, OD tablets, chewable tablets, gums, jellies, gummies, etc. Moreover, the unpleasant taste is difficult to detect even in uncoated solid formulations and semi-solid formulations. Moreover, even in tablets it can inhibit the phenomenon whereby the formulation turns black in the production step, and can therefore provide tablets of high commercial value.

The dose of the inventive oral pharmaceutical formulation is preferably an amount that allows oral use of 100-2400 mg/day, more preferably 120-1200 mg/day, and particularly preferably 390-1200 mg/day of component (A), calculated as free form ibuprofen. Also, it is preferably an amount that allows the oral use of component (A) at 60-800 mg per administration, more preferably 60-400 mg per administration, even more preferably 70-400 mg per administration, and particularly preferably 130-400 mg per administration, calculated as free form ibuprofen.

Also, it is preferably an amount that allows the oral use of magnesium oxide at 30-1000 mg/day.

### <Production method, method for reducing the unpleasant taste, and method for inhibiting discoloration> (only the method for reducing the unpleasant taste is claimed)

The inventive oral pharmaceutical formulation can be produced by a known method in accordance with the dosage form; it is preferably produced by a method that includes a step wherein (A) ibuprofen or salt thereof or solvate of these and (B) magnesium oxide are incorporated into the same composition, such that the mass ratio [(B)/(A)] of component (A) and component (B) in the oral pharmaceutical formulation is 0.2-1.5 (hereafter referred to as step 1). According to this method, an oral pharmaceutical formulation in which the unpleasant taste of ibuprofen is reduced can easily be produced. Furthermore, when producing a solid oral formulation, it can inhibit the phenomenon whereby the formulation turns black in the production step.

### Step 1

In step 1, the amounts of components (A) and (B) used should be adjusted so that the mass ratio [(B)/(A)] of component (A) and component (B) in the target inventive oral pharmaceutical formulation is 0.2-1.5. The order in which components (A) and (B) are incorporated into the composition does not matter.

From the viewpoint of homogenization, reducing the unpleasant taste and inhibiting discoloration in the production step, step 1 is preferably a step wherein components (A) and (B), and any other necessary components (the abovementioned drugs and pharmaceutical additives) are mixed, such that the mass ratio [(B)/(A)] of component (A) and component (B) in the oral pharmaceutical formulation is 0.2-1.5. Specifically, it includes a technique for obtaining a mixture by mixing components (A) and (B) and any other necessary components, and a technique for mixing components (A) and (B) and any other necessary components and kneading the resulting mixture with a solvent such as water or aqueous alcohol as necessary, and granulating by a common method to obtain a granular substance.

Said aqueous alcohol preferably has an alcohol content of no more than 30% by mass. The alcohol is preferably a lower alcohol such as ethanol or isopropanol. Examples of the water used as solvent are purified water, ion-exchanged water, etc.

The abovementioned mixing and kneading can, for example, be performed using a stirring-type mixer or the like. The mixing is preferably performed at 20-1000 rpm for 0.5-10 minutes. The kneading is preferably performed for 0.5-10 minutes.

Said granulation may be performed by a wet granulation method or by a dry granulation method, but preferably a wet granulation method.

Examples of the wet granulation method include a stirring granulation method, fluidized bed granulation method, extrusion granulation method or the like; of these, the extrusion granulation method is preferred.

If the composition obtained in step 1 is a granular substance, its mean particle size is preferably 50-1000 µm, more preferably 100-500 µm. Said mean particle size can be measured by the sieving method.

It should be noted that the mass ratio [(B)/(A)] of component (A) and component (B) in the composition obtained in step 1 is preferably the same as the mass ratio [(B)/(A)] of component (A) and component (B) in the target inventive oral pharmaceutical formulation. Also, the composition obtained in step 1 is preferably a solid.

### Step 2

The inventive production method, as a method for reducing the unpleasant taste and a method for inhibiting discoloration, preferably also comprises a step wherein the composition obtained in step 1 is dried (hereafter referred to as step 2). The inclusion of such a step 2 can further reduce the unpleasant taste of ibuprofen.

Specific examples of techniques for the drying treatment in step 2 include thermal drying, freeze-drying, low-pressure drying, vacuum drying, aeration drying, spray drying, etc. Of these, from the viewpoint of reducing the unpleasant taste, thermal drying is particularly preferred. When thermal drying is employed, the heating temperature should be below the melting point of the respective components in the composition; from the viewpoint of reducing the unpleasant taste it is preferably 50-75°C, more preferably 52.5-72.5°C, even more preferably 55-70°C, even more preferably 57.5-67.5°C, and particularly preferably 60-67.5°C.

The duration of the drying treatment is, from the viewpoint of reducing the unpleasant taste, preferably 0.5-120 hours, more preferably 0.5-96 hours, even more preferably 0.5-72 hours, even more preferably 0.5-48 hours, even more preferably 0.5-24 hours, and particularly preferably 0.5-18 hours.

The drying treatment can be performed using, for example, a box dryer and fluidized bed granulator, low-pressure dryer, vacuum dryer, ventilation dryer, spray dryer, or the like.

Also, if steps 1 and 2 yield a granular substance, after step 1 and/or step 2 it may be subjected to grinding, sieving, spheronization treatment using Marumerizer, coating with saccharide or polymer or the like, etc. Also, if steps 1 and 2 yield a granular substance, the resulting granular substance can be used without further modification as granules, fine granules or powder. It is also possible to obtain tablets by tabletting the granular substance in accordance with a common method, and it is possible to obtain capsules by filling the granular substance into capsules in accordance with a common method.

Furthermore, a liquid formulation can be obtained by mixing the composition obtained in steps 1 and 2 (specifically, the solid composition) with water. This liquid formulation (liquid composition) may also be heat-treated. This heat treatment can further reduce the unpleasant taste of ibuprofen.

The heating temperature for the liquid composition is, from the viewpoint of reducing the unpleasant taste, preferably 50-100°C, more preferably 52.5-97.5°C, even more preferably 55-95°C, even more preferably 57.5-92.5°C, and particularly preferably 60-90°C.

Also, from the viewpoint of reducing the unpleasant taste, the duration of the heating of the liquid composition is preferably 0.5-120 hours, more preferably 0.5-96 hours, even more preferably 0.5-72 hours, even more preferably 0.5-48 hours, even more preferably 0.5-24 hours, and particularly preferably 0.5-18 hours.

The inventive oral pharmaceutical formulations obtained as described above are such that the unpleasant taste of ibuprofen is reduced. Moreover, in the case of solid oral formulations, they are obtained without turning black in the production step.

The Inventive oral pharmaceutical formulations can therefore fully exhibit the anti-inflammatory analgesic effect of ibuprofen, and are extremely useful as antipyretic analgesics, cold medicines, sleep-improving drugs, neuropsychiatric drugs, etc.

### Examples

The present invention is described in detail below with reference to examples, but the present invention is not limited to these examples. In the test examples, the ibuprofen used was Ibuprofen 25 manufactured by BASF; the magnesium oxide used was heavy type and light type magnesium oxide manufactured by Kyowa Chemical Industry Co., Ltd.; and the magnesium chloride hexahydrate used was Special Grade reagent manufactured by Wako Pure Chemical Corporation.

### Example 1

Ibuprofen was passed completely through a 30 mesh, and the resulting ibuprofen plus magnesium oxide (example 1) or magnesium chloride hexahydrate (comparative example 1) were weighed into a glass bottle in the respective proportions shown in Table 1 below. Next, the glass bottle was sealed using a metal cap, and mixing in a vortex mixer was performed for 3 minutes.

Immediately after the mixing, a sample equivalent to 200 mg ibuprofen was placed in the mouth, held in the mouth for 3 minutes without swallowing, and subjected to sensory evaluation, over time, of the unpleasant taste of ibuprofen (irritation and bitterness). For the sample used in reference example 1 (ibuprofen passed completely through a 30 mesh) shown in Table 1, sensory evaluation was performed in accordance with the operations described above using the evaluation criteria below, and the evaluations of example 1 and comparative example 1 were subjected to relative evaluation based on the evaluation result for the reference example 1 sample. The results are shown in Table 1.

### Evaluation criteria

AA: No unpleasant taste was detected.
A: An unpleasant taste was just about detected, but it was not so bothersome.
B: There was an unpleasant taste and it was very bothersome.
C: It was unacceptably unpleasant.

**[Table 1]**

| | | Reference example 1 | Example 1 | Comparative example 1 |
|---|---|---|---|---|
| Ibuprofen | | 200 mg | 200 mg | 200 mg |
| MgO (heavy magnesium oxide) | | 0 mg | 250 mg | 0 mg |
| MgCl₂⁻6H₂O | | 0 mg | 0 mg | 106.5 mg (50 mg calculated as anhydride) |
| [(B)/(A)] | | - | 1.25 | - |
| Time | 10 seconds | AA | AA | A |
| | 20 seconds | A | A | A |
| | 30 seconds | B | A | B |
| | 45 seconds | C | A | C |
| | 1 minute | C | B | C |
| | 2 minutes | C | C | C |
| | 3 minutes | C | C | C |

As shown in Table 1, the mixture of ibuprofen and magnesium chloride hexahydrate (comparative example 1) was the same as ibuprofen (reference example 1) in that both exhibited a distinctly unpleasant taste after having been held in the mouth for 30 seconds.

On the other hand, the mixture in which the mass ratio [(B)/(A)] of the ibuprofen and magnesium oxide was 1.25 (example 1) did not exhibit such an unpleasant taste even after having been held in the mouth for 45 seconds.

### Example 2

Ibuprofen was passed completely through a 30 mesh, and the resulting ibuprofen plus magnesium oxide (examples 2-7 and comparative example 4) or magnesium chloride hexahydrate (comparative example 3) were weighed into a glass bottle in the respective proportions shown in Table 2 below. Next, the glass bottle was sealed using a metal cap, and mixing in a vortex mixer was performed for 3 minutes. The sealed bottle was then stored at 65°C for 15 hours, then allowed to cool; thus samples for examples 2-7 and comparative examples 3-4 were obtained.

Also, ibuprofen was passed completely through a 30 mesh, and 200 mg of the resulting ibuprofen was introduced into a glass bottle which was then sealed using a metal cap, and the sealed bottle was stored at 65°C for 15 hours, then allowed to cool to obtain the sample for comparative example 2.

Said samples for examples 2-7 and comparative examples 2-4 were subjected to sensory evaluation of the unpleasant taste of ibuprofen (irritation and bitterness) in accordance with the operations and evaluation criteria used in test example 1 (relative evaluation based on the evaluation result for the reference example 1 sample). The results are shown in Table 2.

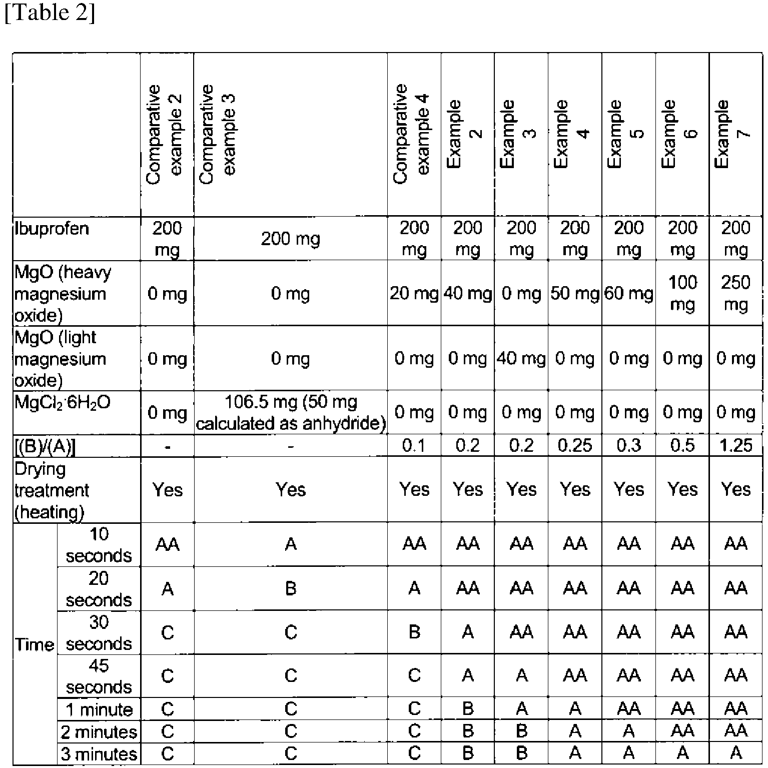

As shown in Table 2, the mixture of ibuprofen and magnesium chloride hexahydrate (comparative example 3) exhibited a distinctly unpleasant taste after having been held in the mouth for 20 seconds. Also, the mixture having a mass ratio [(B)/(A)] lower than 0.2 (comparative example 4) exhibited a distinctly unpleasant taste after having been held in the mouth for 30 seconds.

On the other hand, the mixtures having mass ratios [(B)/(A)] in the range of 0.2-1.25 (examples 2-7) did not exhibit such an unpleasant taste even after having been held in the mouth for 45 seconds.

Also, as is clear from Tables 1 and 2, when the ibuprofen was subjected to the drying treatment (comparative example 2) or the mixture of ibuprofen and magnesium chloride hexahydrate was subjected to the drying treatment (comparative example 3), the unpleasant taste was no lower than in the substances that had not been subjected to the drying treatment (reference example 1, comparative example 1).

On the other hand, when the mixture of ibuprofen and magnesium oxide was subjected to the drying treatment (example 7), the unpleasant taste was even lower than in the substance that had not been subjected to the drying treatment (example 1).

### Example 3

Ibuprofen, magnesium oxide and low-substituted hydroxypropyl cellulose (L-HPC (LH31) manufactured by Shin-Etsu Chemical Co., Ltd.) were weighed out in the respective proportions shown in Table 3 below, to a total of 50 g. This was introduced into a Mechanomill and mixed at 900 rpm for 3 minutes. Next, purified water in an amount shown in Table 3 below was added, kneading was performed for 3 minutes, and then extrusion-granulation was performed. The resulting granular substance was dried using a box dryer (overnight at 65°C). It was sized to obtain the granules of examples 8-12 and comparative example 5 (16-60 mesh).

Granules equivalent to 200 mg ibuprofen were subjected to sensory evaluation of the unpleasant taste of ibuprofen (irritation and bitterness) in accordance with the operations and evaluation criteria used in test example 1 (relative evaluation based on the evaluation result for the reference example 1 sample). The results are shown in Table 3.

**[Table 3]**

| | | Comparative example 5 | Example 8 | Example 9 | Example 10 | Example 11 | Example 12 |
|---|---|---|---|---|---|---|---|
| Ibuprofen | | 200 mg | 200 mg | 200 mg | 200 mg | 200 mg | 200 mg |
| MgO (heavy magnesium oxide) | | 35 mg | 40 mg | 50 mg | 75 mg | 100 mg | 250 mg |
| L-HPC (LH31) | | 101 mg | 103 mq | 107 mq | 118 mg | 129 mg | 193 mg |
| Purified water | | (512 mg) | (564 mg) | (534 mg) | (624 mg) | (659 mg) | (1093 mg) |
| [(B)/(A)] | | 0.175 | 0.2 | 0.25 | 0.375 | 0.5 | 1.25 |
| Time | 10 seconds | AA | AA | AA | AA | AA | AA |
| | 20 seconds | A | AA | AA | AA | AA | AA |
| | 30 seconds | B | AA | AA | AA | AA | AA |
| | 45 seconds | B | AA | AA | AA | AA | AA |
| | 1 minute | C | AA | AA | AA | AA | AA |
| | 2 minutes | C | A | AA | AA | AA | AA |
| | 3 minutes | C | A | A | A | AA | AA |

As shown in Table 3, granules wherein the mass ratio [(B)/(A)] was less than 0.2 (comparative example 5) exhibited a distinctly unpleasant taste after having been held in the mouth for 30 seconds.

On the other hand, granules wherein the mass ratio [(B)/(A)] was 0.2-1.25 (examples 8-12) did not exhibit such an unpleasant taste even after having been held in the mouth for 3 minutes.

### Example 4

Sensory evaluation the same as in test example 3 was conducted with the exception that the composition of the granules was modified to the compositions shown in Table 4 and that the granules were evaluated only after having been held in the mouth for 30 seconds and after having been held in the mouth for 1 minute. The results are shown in Table 4.

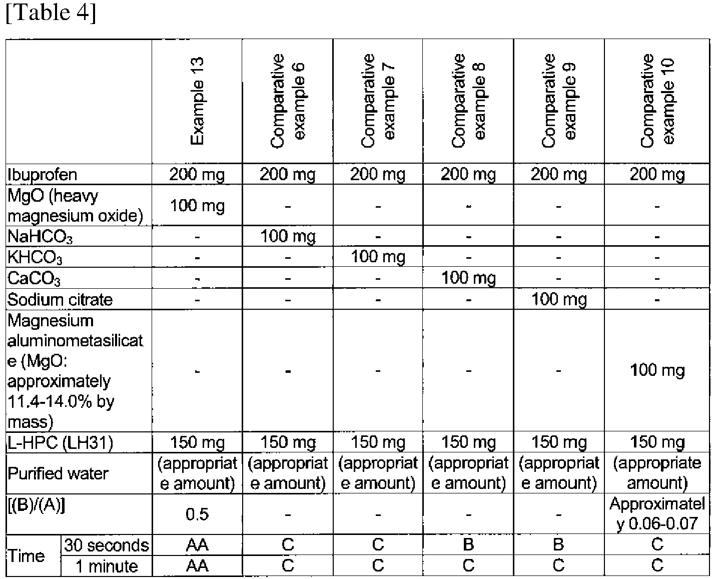

### Example 5

Ibuprofen, magnesium oxide and low-substituted hydroxypropyl cellulose (L-HPC (LH31) manufactured by Shin-Etsu Chemical Co., Ltd.) were weighed out in the respective proportions shown in Table 5 below, to a total of 50 g. This was introduced into a Mechanomill and mixed at 900 rpm for 3 minutes. Next, the appropriate amount of purified water was added and the resulting system was kneaded for 3 minutes, and then extrusion-granulated. The resulting granular substance was dried using a box dryer (overnight at 65°C). It was sized to obtain the granules of example 14 (16-60 mesh).

Granules equivalent to 200 mg ibuprofen were subjected to sensory evaluation of the unpleasant taste of ibuprofen (irritation and bitterness) in accordance with the operations and evaluation criteria used in test example 1 (relative evaluation based on the evaluation result for the reference example 1 sample). The results are shown in Table 5.

**[Table 5]**

| | | Example 14 |
|---|---|---|
| Ibuprofen | | 200 mg |
| MgO (heavy magnesium oxide) | | 300 mg |
| L-HPC (LH31) | | 214 mg |
| Purified water | | (appropriate amount) |
| [(B)/(A)] | | 1.5 |
| Time | 10 seconds | AA |
| | 20 seconds | AA |
| | 30 seconds | AA |
| | 45 seconds | AA |
| | 1 minute | AA |
| | 2 minutes | AA |
| | 3 minutes | A |

As shown in Table 5, granules wherein the mass ratio [(B)/(A)] was 1.5 (example 14) were similar to the granules of examples 8-12 in that they did not exhibit such an unpleasant taste even after having been held in the mouth for 3 minutes.

### Example 6

Ibuprofen, magnesium oxide and low-substituted hydroxypropyl cellulose (L-HPC (LH31) manufactured by Shin-Etsu Chemical Co., Ltd.) were weighed out in the respective proportions shown in Table 6 below, to a total of 50 g. This was introduced into a Mechanomill and mixed at 900 rpm for 3 minutes. Next, the appropriate amount of purified water was added, and the resulting system was kneaded for 3 minutes, and then extrusion-granulated. The resulting granular substance was dried using a box dryer (overnight at 65°C). It was sized to obtain granules (16-60 mesh). Next, a tabletting powder was prepared by adding 1 part by mass of magnesium stearate per 100 parts by mass of the resulting granules, and mixing thoroughly. This was then tabletted using a single-tablet press to obtain uncoated tablets of examples 15-16 and reference examples 2-3 (diameter 10 mm, mass of 1 tablet 400 mg).

The color of the surfaces and sides of the uncoated tablets were confirmed visually immediately after production. Those that were changed into a different color (black) from that of the granular substance were evaluated as having "blackened", and those that exhibited no change in color from the granular substance were evaluated as "not blackened". The results are shown in Table 6.

**[Table 6]**

| | Example 15 | Example 16 | Reference example 2 | Reference Example 3 |
|---|---|---|---|---|
| Ibuprofen | 200 mg | 200 mg | 200 mg | 200 mg |
| MgO (heavy magnesium oxide) | 40 mg | 300 mg | 500 mg | 900 mg |
| L-HPC (LH31) | 103 mg | 214 mg | 300 mg | 471 mg |
| Purified water | (appropriate amount) | (appropriate amount) | (appropriate amount) | (appropriate amount) |
| [(B)/(A)] | 0.2 | 1.5 | 2.5 | 4.5 |
| Color of tablet | Not blackened | Not blackened | Blackened | Blackened |

### Example 7

Granules of example 11, prepared in test example 3, were weighed out to obtain an amount equivalent to 400 mg ibuprofen. These granules will be referred to as "granules of example 17".

Granules of example 17 were placed in the mouth, held in the mouth for 3 minutes without swallowing, and subjected to sensory evaluation, over time, of the unpleasant taste of ibuprofen (irritation and bitterness). For the reference example 4 sample (ibuprofen passed completely through a 30 mesh) shown in Table 7, sensory evaluation of an amount equivalent to 400mg ibuprofen was performed in accordance with the operations as described above, using the following evaluation criteria, and the evaluations for example 17 were subjected to relative evaluation based on the evaluation result for the reference example 4 sample. The results are shown in Table 7.

Evaluation criteria
AA: No unpleasant taste was detected.
A: An unpleasant taste was just about detected, but it was not so bothersome.
B: There was an unpleasant taste and it was very bothersome.
C: It was unacceptably unpleasant.

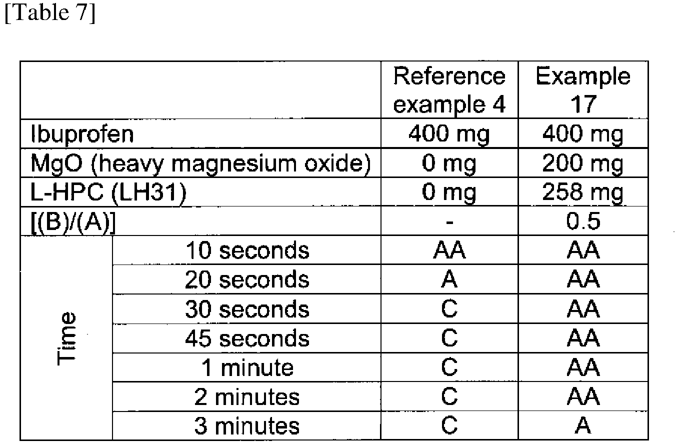

As shown in Table 7, the granules wherein the mass ratio [(B)/(A)] was 0.5 did not have such an unpleasant taste even after an amount equivalent to 400 mg ibuprofen had been held in the mouth for 3 minutes.

### Example 8

### - Preparation of liquid agents -

The liquid agents of reference example 5, example 18 and comparative example 11 were prepared in accordance with the procedures below.

### Reference example 5 liquid agent

Ibuprofen was passed completely through a 30 mesh and the resulting ibuprofen was weighed into a glass bottle in the proportion shown in Table 8 below. Next, the glass bottle was sealed using a metal cap, mixing in a vortex mixer was performed for 3 minutes, and then purified water was added in the proportion shown in Table 8 below, and shaking was performed to obtain a suspension, which was used as liquid agent reference example 5.

### Example 18 liquid agent

Ibuprofen was passed completely through a 30 mesh and then the resulting ibuprofen plus magnesium oxide were weighed into a glass bottle in the proportions shown in Table 8 below. Next, the glass bottle was sealed using a metal cap, mixing in a vortex mixer was performed for 3 minutes, and then the total amount was transferred onto a plastic tray. The plastic tray was then stored at 65°C for 15 hours; after having been allowed to cool, the resulting substance was crushed using a mortar, and then purified water was added in the proportion shown in Table 8 below; shaking was performed to obtain a suspension, which was used as example 18 liquid agent.

### Comparative example 11 liquid agent

Ibuprofen was passed completely through a 30 mesh and then the resulting ibuprofen plus magnesium chloride hexahydrate were weighed into a glass bottle in the proportions shown in Table 8 below. Next, the glass bottle was sealed using a metal cap, mixing in a vortex mixer was performed for 3 minutes, then purified water was added in the proportion shown in Table 8 below, and shaking was performed to obtain a suspension which was used as comparative example 11 liquid agent.

### - Evaluation of liquid agents -

For the example 18 and comparative example 11 liquid agents respectively, 10 g of the liquid agent (equivalent to 400 mg ibuprofen) were introduced into the mouth, held in the mouth for 3 minutes without swallowing, and subjected to sensory evaluation, over time, of the unpleasant taste of ibuprofen (irritation and bitterness). For 10 g of reference example 5 liquid agent, sensory evaluation was performed in accordance with the operations described above, using the following evaluation criteria, and the evaluations for example 18 and comparative example 11 were subjected to relative evaluation based on the evaluation result for the reference example 5 liquid agent. The results are shown in Table 8.

### Evaluation criteria

AA: No unpleasant taste was detected.
A: An unpleasant taste was just about detected, but it was not so bothersome.
B: There was an unpleasant taste and it was very bothersome.
C: It was unacceptably unpleasant.

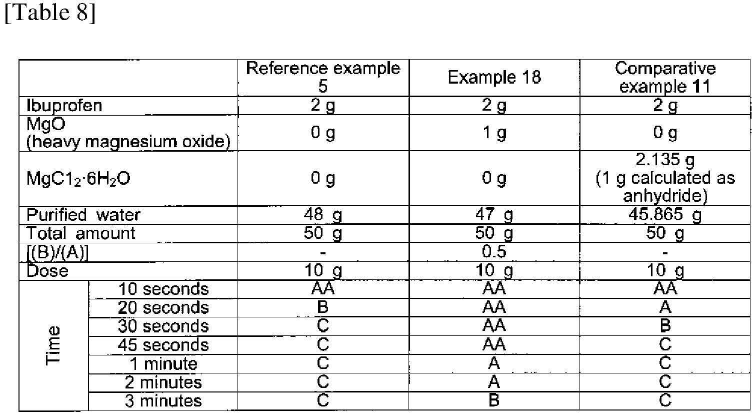

### Example 9

### - Preparation of liquid agents -

The reference example 6, example 19 and example 20 liquid agents were prepared in accordance with the procedures below.

### Reference example 6 liquid agent

The reference example 5 liquid agent was sealed and heated at 60°C for 2 hours to obtain reference example 6 liquid agent.

### Example 19 liquid agent

Ibuprofen was passed completely through a 30 mesh and then the resulting ibuprofen plus magnesium oxide were weighed into a glass bottle in the proportions shown in Table 9 below. Next, the glass bottle was sealed using a metal cap, and mixing in a vortex mixer was performed for 3 minutes. Purified water was then added in the proportion shown in Table 9 below, and shaking was performed to obtain a suspension, which was then heated at 60°C for 2 hours, while still sealed, to obtain example 19 liquid agent.

### Example 20 liquid agent

The example 18 liquid agent was sealed and heated at 60°C for 2 hours to obtain example 20 liquid agent.

### - Evaluation of liquid agents -

Using 10 g of the liquid agents of reference example 6 and examples 19-20 respectively, sensory evaluation of the unpleasant taste of ibuprofen (irritation and bitterness) was performed in accordance with the operations and evaluation criteria used in example 8 (relative evaluation based on the evaluation result for the reference example 5 liquid agent). The results are shown in Table 9.

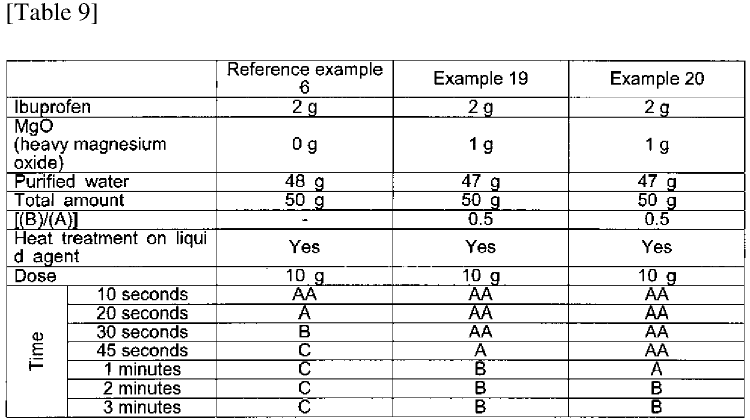

As shown in Tables 8-9, the pharmaceutical formulations wherein the mass ratio [(B)/(A)] was 0.2-1.5 were such that the unpleasant taste was difficult to detect even when the dosage form was a liquid agent.

### Formulation example 1: powder

Ibuprofen (20 g) (Ibuprofen 25 manufactured by BASF), 5 g magnesium oxide (heavy type manufactured by Kyowa Chemical Industry Co., Ltd.) and 20 g low-substituted hydroxypropyl cellulose (L-HPC (LH31) manufactured by Shin-Etsu Chemical Co., Ltd.) were weighed out. These were introduced into a Mechanomill (MM-20 manufactured by OKADA SEIKO CO.,LTD.) and mixed at 900 rpm for 3 minutes. Next, the resulting mixture was heated for 12 hours in a box dryer set at a temperature of 65°C. It was then sieved through a number 30 sieve of mesh size 500 µm, and the powder that passed through the number 30 sieve was obtained. To 22.5 g of the resulting powder, 25.75 g d-mannitol (Mannit P manufactured by Mitsubishi Corporation Life Sciences Limited), 0.25 g light anhydrous silicic acid (Adsolider-101 manufactured by Fuji Silysia), 0.5 g aspartame (manufactured by Ajinomoto Co., Inc.), 0.5 g acesulfame potassium (manufactured by MC Food Specialties) and 0.5 g magnesium stearate (botanical manufactured by TAIHEI CHEMICAL INDUSTRIAL CO., LTD.) were added and mixed; and then aluminum heat seal filling was performed, such that each pack contained 1000 mg powder.

### Formulation example 2: granules

Ibuprofen (300 g) (Ibuprofen 25 manufactured by BASF), 300 g magnesium oxide (heavy type manufactured by Kyowa Chemical Industry Co., Ltd.) and 450 g low-substituted hydroxypropyl cellulose (L-HPC (LH31) manufactured by Shin-Etsu Chemical Co., Ltd.) were weighed out. These were introduced into a vertical granulator (FM-VG-10 manufactured by Powrex) and mixed at a blade rotation speed of 450 rpm and a chopper rotation speed of 2000 rpm for 3 minutes. Next, 1800 g purified water was added and the resulting system was kneaded for 3 minutes, then extrusion-granulated using a screen diameter of 0.6 mm (TDG-80A manufactured by Dalton). The resulting wet granules were introduced into a fluidized bed dryer (FLO-2 manufactured by Freund Corporation) and dried at an air supply temperature of 85°C. These were sized (QC-U10 manufactured by Powrex) to obtain granules. To 700 g of the resulting granules, 361.5 g d-mannitol (Mannit P manufactured by Mitsubishi Corporation Life Sciences Limited), 5.5 g light anhydrous silicic acid (Adsolider-101 manufactured by Fuji Silysia), 11 g aspartame (manufactured by Ajinomoto Co., Inc.), 11 g acesulfame potassium (manufactured by MC Food Specialties) and 11 g magnesium stearate (botanical manufactured by TAIHEI CHEMICAL INDUSTRIAL CO., LTD.) were added and mixed; and then aluminum heat seal filling was performed, such that each pack contained 1100 mg powder.

### Formulation example 3: uncoated tablet

Ibuprofen (500 g) (Ibuprofen 25 manufactured by BASF), 250 g magnesium oxide (heavy type manufactured by Kyowa Chemical Industry Co., Ltd.) and 250 g low-substituted hydroxypropyl cellulose (L-HPC (LH31) manufactured by Shin-Etsu Chemical Co., Ltd.) were weighed out. These were introduced into a vertical granulator (FM-VG-10 manufactured by Powrex) and mixed at a blade rotation speed of 450 rpm and a chopper rotation speed of 2000 rpm for 3 minutes. Next, 1050 g purified water was added and the resulting system was kneaded for 3 minutes, and then extrusion-granulated using a screen diameter of 0.6 mm (TDG-80A manufactured by Dalton). The resulting wet granules were introduced into a fluidized bed dryer (FLO-2 manufactured by Freund Corporation) and dried at an air supply temperature of 85°C. These were sized (QC-U10 manufactured by Powrex) to obtain granules. To 800 g of the resulting granules, 370 g crystalline cellulose (Ceolus PH-F20JP manufactured by Asahi Kasei), 6 g light anhydrous silicic acid (Adsolider-101 manufactured by Fuji Silysia), 12 g talc (Lisblanc manufactured by Kihara Kasei) and 12 g magnesium stearate (botanical manufactured by TAIHEI CHEMICAL INDUSTRIAL CO., LTD.) were added and mixed; and then tabletting was performed to obtain uncoated tablets where each tablet weighed 600 mg and had a diameter of 12 mm.

### Formulation example 4: orally disintegrating tablet

Ibuprofen (600 g) (Ibuprofen 25 manufactured by BASF), 180 g magnesium oxide (heavy type manufactured by Kyowa Chemical Industry Co., Ltd.) and 240 g low-substituted hydroxypropyl cellulose (L-HPC (LH31) manufactured by Shin-Etsu Chemical Co., Ltd.) were weighed out. These were introduced into a vertical granulator (FM-VG-10 manufactured by Powrex) and mixed at a blade rotation speed of 450 rpm and a chopper rotation speed of 2000 rpm for 3 minutes. Next, 1000 g purified water was added and the resulting system was kneaded for 3 minutes, and then extrusion-granulated using a screen diameter of 0.6 mm (TDG-80A manufactured by Dalton). The resulting wet granules were introduced into a fluidized bed dryer (FLO-2 manufactured by Freund Corporation) and dried at an air supply temperature of 85°C. These were sized (QC-U10 manufactured by Powrex) to obtain granules. To 680 g of the resulting granules, 270 g crystalline cellulose (Ceolus PH-F20JP manufactured by Asahi Kasei), 100 g crospovidone (CL-F manufactured by BASF), 6 g light anhydrous silicic acid (Adsolider-101 manufactured by Fuji Silysia), 11 g aspartame (manufactured by Ajinomoto Co., Inc.), 11 g acesulfame potassium (manufactured by MC Food Specialties), 11 g talc (Lisblanc manufactured by Kihara Kasei) and 11 g magnesium stearate (botanical manufactured by TAIHEI CHEMICAL INDUSTRIAL CO., LTD.) were added and mixed; and then tabletting was performed to obtain orally disintegrating tablets where each tablet weighed 550 mg and had a diameter of 12 mm.

### Formulation example 5: chewable tablet

Ibuprofen (500 g) (Ibuprofen 25 manufactured by BASF), 250 g magnesium oxide (heavy type manufactured by Kyowa Chemical Industry Co., Ltd.) and 250 g low-substituted hydroxypropyl cellulose (L-HPC (LH31) manufactured by Shin-Etsu Chemical Co., Ltd.) were weighed out. These were introduced into a vertical granulator (FM-VG-10 manufactured by Powrex) and mixed at a blade rotation speed of 450 rpm and a chopper rotation speed of 2000 rpm for 3 minutes. Next, 1100 g purified water was added and the resulting system was kneaded for 3 minutes, and then extrusion-granulated using a screen diameter of 0.6 mm (TDG-80A manufactured by Dalton). The resulting wet granules were introduced into a fluidized bed dryer (FLO-2 manufactured by Freund Corporation) and dried at an air supply temperature of 85°C. These were sized (QC-U10 manufactured by Powrex) to obtain granules. To 800 g of the resulting granules, 200 g crystalline cellulose (Ceolus PH-F20JP manufactured by Asahi Kasei), 246 g d-mannitol (Granutol R manufactured by Freund Corporation), 6 g light anhydrous silicic acid (Adsolider-101 manufactured by Fuji Silysia), 12 g aspartame (manufactured by Ajinomoto Co., Inc.), 12 g acesulfame potassium (manufactured by MC Food Specialties), 12 g talc (Lisblanc manufactured by Kihara Kasei) and 12 g magnesium stearate (botanical manufactured by TAIHEI CHEMICAL INDUSTRIAL CO., LTD.) were added and mixed; and then tabletting was performed to obtain chewable tablets where each tablet weighed 650 mg and had a diameter of 12 mm.

## Claims

1. A method for reducing the unpleasant taste of ibuprofen in oral pharmaceutical formulations that contain ibuprofen or salt thereof or solvate of these, comprising a step of incorporating (A) ibuprofen or salt thereof or solvate of these, and (B) magnesium oxide into the same composition, such that the mass ratio [(B)/(A)] of component (A) and component (B) in the oral pharmaceutical formulation is 0.2-1.5.

2. The method as claimed in claim 1, further comprising a step of drying the composition obtained in the abovementioned step.

3. The method as claimed in claim 2, wherein the drying step comprises thermal drying at 50-75°C.

4. The method as claimed in any one of claims 1 to 3, wherein the mass ratio [(B)/(A)] is 0.25-1.5.

5. The method as claimed in any one of claims 1 to 4, wherein the mass ratio [(B)/(A)] is 0.3-1.5.

6. The method as claimed in any one of claims 1 to 5, wherein the mass ratio [(B)/(A)] is 0. 4-1. 5.

7. The method as claimed in any one of claims 1 to 6, wherein the dosage form of the oral pharmaceutical formulation is a solid formulation or semi-solid formulation.

8. The method as claimed in claim 7, wherein the dosage form is granules, fine granules, powder, uncoated tablet, OD tablet, chewable tablet, gum, jelly or gummy.

9. The method as claimed in any one of claims 1 to 6, wherein the dosage form of the oral pharmaceutical formulation is a suspension.

## Patentansprüche

1. Verfahren zum Reduzieren des unangenehmen Geschmacks von Ibuprofen in oralen pharmazeutischen Formulierungen, die Ibuprofen oder Salz davon oder Solvat dieser enthalten, das einen Schritt des Einbindens von (A) Ibuprofen oder Salz davon oder Solvat dieser, und (B) Magnesiumoxid in die gleiche Zusammensetzung derart umfasst, dass das Massenverhältnis [(B)/(A)] von Bestandteil (A) und Bestandteil (B) in der oralen pharmazeutischen Formulierung 0,2-1,5 beträgt.

2. Verfahren nach Anspruch 1, das weiter einen Schritt des Trocknens der in dem oben genannten Schritt erhaltenen Zusammensetzung umfasst.

3. Verfahren nach Anspruch 2, wobei der Trocknungsschritt thermisches Trocknen bei 50-75 °C umfasst.

4. Verfahren nach einem der Ansprüche 1 bis 3, wobei das Massenverhältnis [(B)/(A)] 0,25-1,5 beträgt.

5. Verfahren nach einem der Ansprüche 1 bis 4, wobei das Massenverhältnis [(B)/(A)] 0,3-1,5 beträgt.

6. Verfahren nach einem der Ansprüche 1 bis 5, wobei das Massenverhältnis [(B)/(A)] 0,4-1,5 beträgt.

7. Verfahren nach einem der Ansprüche 1 bis 6, wobei es sich bei der Dosierungsform der oralen pharmazeutischen Formulierung um eine feste Formulierung oder halbfeste Formulierung handelt.

8. Verfahren nach Anspruch 7, wobei es sich bei der Dosierungsform um Granulat, Feingranulat, Pulver, unbeschichtete Tablette, OD-Tablette, kaubare Tablette, Kaugummi, Gelee oder Gummi handelt.

9. Verfahren nach einem der Ansprüche 1 bis 6, wobei es sich bei der Dosierungsform der oralen pharmazeutischen Formulierung um eine Suspension handelt.

## Revendications

1. Procédé de réduction du goût déplaisant de l'ibuprofène dans des formulations pharmaceutiques orales contenant de l'ibuprofène ou un sel de celui-ci ou un solvate de ceux-ci, comprenant une étape d'incorporation (A) d'ibuprofène ou d'un sel de celui-ci ou d'un solvate de ceux-ci, et (B) d'oxyde de magnésium dans la même composition, de telle sorte que le rapport en masse [(B)/(A)] du composant (A) et du composant (B) dans la formulation pharmaceutique soit de 0,2 à 1,5.

2. Procédé selon la revendication 1, comprenant en outre une étape de séchage de la composition obtenue à l'étape susmentionnée.

3. Procédé selon la revendication 2, dans lequel l'étape de séchage comprend un séchage thermique à 50 à 75 °C.

4. Procédé selon l'une quelconque des revendications 1 à 3, dans lequel le rapport en masse [(B)/(A)] est de 0,25 à 1,5.

5. Procédé selon l'une quelconque des revendications 1 à 4, dans lequel le rapport en masse [(B)/(A)] est de 0,3 à 1,5.

6. Procédé selon l'une quelconque des revendications 1 à 5, dans lequel le rapport en masse [(B)/(A)] est de 0,4 à 1,5.

7. Procédé selon l'une quelconque des revendications 1 à 6, dans lequel la forme posologique de la formulation pharmaceutique orale est une formulation solide ou une formulation semi-solide.

8. Procédé selon la revendication 7, dans lequel la forme posologique est des granules, des granules fins, une poudre, un comprimé non enrobé, un comprimé OD, un comprimé à croquer, une gomme, une gelée ou une gomme à mâcher.

9. Procédé selon l'une quelconque des revendications 1 à 6, dans lequel la forme posologique de la formulation pharmaceutique orale est une suspension.
